# DEMANDE DE BREVET EUROPEEN

(11) **EP 1 166 768 A1**
(43) Date de publication de la demande: **02.01.2002**
(21) Numéro de dépôt: 01401343.7
(22) Date de dépôt: 22.05.2001
(51) Int. Cl.: A61K 7/48

(54) **Utilisation d'ergothionéine et/ou de ses dérivés comme agent anti-glycation**

(30) Priorité: 26.06.2000 FR 0008158
(71) Demandeur: L'OREAL, 75008 Paris (FR)
(72) Inventeur: Pelletier, Pascale, 92160 Antony (FR); Pageon, Hervé, 92800 Puteaux (FR)
(74) Mandataire: Renard, Emmanuelle

(57) **Abrégé**

L'invention se rapporte à l'utilisation d'ergothionéine et/ou de ses dérivés dans une composition cosmétique, et/ou pour la fabrication d'une composition dermatologique, destinée à prévenir ou traiter la perte de tonicité de la peau, en particulier due à l'âge, ainsi qu'à son utilisation dans une composition cosmétique destinée à prévenir ou traiter l'aspect peau d'orange.

Cette composition peut notamment être à visée anti-âge ou amincissante.

Il a en effet été découvert que l'ergothionéine présentait la propriété de diminuer la glycation des protéines, en particulier des protéines du derme telles que le collagène, responsable de la rigidification des tissus.

## Description

L'invention se rapporte à l'utilisation d'ergothionéine et/ou de ses dérivés dans une composition cosmétique, en tant qu'agent pour diminuer la glycation des protéines de la peau, en particulier pour prévenir ou traiter les signes de vieillissement de la peau et/ou l'aspect peau d'orange et pour amincir et/ou affiner la silhouette et/ou les contours du visage.

La glycation est un processus non enzymatique faisant intervenir un ose (glucose ou ribose) qui réagit selon la réaction de Maillard avec un groupement aminé d'un résidu d'acide aminé (comme par exemple la lysine), particulièrement un résidu d'acide aminé d'une protéine, pour former une base de Schiff. Celle-ci, après un réarrangement moléculaire dit d'Amadori, peut conduire, par une succession de réactions, à un pontage, particulièrement intramoléculaire comme par exemple de type pentosidine.

Ce phénomène se caractérise par l'apparition de produits de glycation dont la teneur augmente de façon régulière en fonction de l'âge. Les produits de glycation sont par exemple la pyrraline, la carboxyméthyl-lysine, la pentosidine, la crossline, la N^{ε}(2-carboxyéthyl)-lysine (CEL), la glyoxal-lysine dimer (GOLD), la méthylglyoxal-lysine dimer (MOLD), la 3DG-ARG imidazolone, les versperlysines A, B, C, la thréosidine ou encore les produits finaux de glycosylation avancée (ou AGEs).

La glycation des protéines est donc un phénomène universel, bien connu au niveau de la peau, particulièrement au niveau de sa composante dermique, et principalement au niveau des fibres de collagène. La glycation du collagène augmente en effet de façon régulière avec l'âge, entraînant une augmentation régulière de la teneur de la peau en produits de glycation.

Sans vouloir introduire une quelconque théorie du vieillissement de la peau, il faut noter que d'autres modifications du collagène qui pourraient également être une conséquence de la glycation, comme une diminution de la dénaturation par la chaleur, une augmentation de la résistance à la digestion enzymatique et une augmentation des pontages intermoléculaires, ont pu être mises en évidence au cours du vieillissement de la peau ( Tanaka S. et col., 1988, J. Mol. Biol., 203, 495-505 ; Takahashi M. et col., 1995, Analytical Biochemistry, 232, 158-162 ). De plus, des modifications dues à la glycation de certains constituants de la membrane basale comme le collagène IV, la laminine et la fibronectine ont pu être mises en évidence (Tarsio JF. et col. , 1985, Diabetes, 34, 477-484 ; Tarsio JF. et col, 1988, Diabetes, 37, 532-539 ; Sternberg M. et col., 1995, C. R. Soc. Biol., 189, 967-985 ).

Ainsi, on comprend qu'au cours du vieillissement de la peau les propriétés physico-chimiques du collagène se modifient et ce dernier devient plus difficilement soluble et plus difficilement dégradable. Il s'ensuit une rigidification des tissus conduisant essentiellement à une perte de tonicité de la peau.

Outre ses effets sur le vieillissement de la peau, la glycation intervient dans l'aspect "peau d'orange" caractéristique de la cellulite. Dans la cellulite, en effet, la glycation du collagène constituant la plus grande part des travées conjonctives entraîne une rigidification des tissus qui emprisonnent alors les globules graisseux. La peau présente ainsi une succession de bosses formées d'amas graisseux et de creux formés des travées conjonctives rigidifiées, caractéristiques de l'aspect "peau d'orange".

On comprend donc l'importance qui existe à disposer de produits qui diminuent voire inhibent le phénomène de glycation des protéines.

On connaît différents produits capable d'inhiber cette réaction de glycation : l'aminoguanidine (qui est l'inhibiteur le plus connu), la taurine, certaines vitamines (B1, B6) et les dérivés thiazolium.

La demanderesse a maintenant découvert de manière surprenante et inattendue que l'ergothionéine et ses dérivés présentent la propriété de diminuer voire inhiber le phénomène de glycation des protéines et d'agir ainsi, d'une part, sur la perte de tonicité de la peau due à l'âge et, d'autre part, sur l'aspect "peau d'orange". En outre, l'effet anti-glycant de l'ergothionéine pourrait éventuellement trouver une application intéressante dans des produits d'amincissement du visage qui visent à redessiner les contours du visage déformés par l'âge.

L'ergothionéine a déjà été décrite comme inhibiteur de la mélanogénèse [(Motohashi, N. et al., Chem. Pharm. Bull., 39(1), 142-5 (1991) et JP-63 008 335] et comme piégeur de radicaux libres [Whiteman M. et al., FEBS Lett., 414(3), 497-500 (1997)]. Toutefois, à la connaissance de la Demanderesse, il n'a encore jamais été suggéré qu'elle pouvait avoir des propriétés d'inhibition de la glycation.

L'invention a donc pour objet l'utilisation de l'ergothionéine et/ou de ses dérivés dans une composition cosmétique, en tant qu'agent pour diminuer la glycation des protéines de la peau. Elle a également pour objet l'utilisation de l'ergothionéine et/ou de ses dérivés pour la fabrication d'une composition dermatologique destinée à diminuer la glycation des protéines de la peau.

De préférence, les protéines de la peau sont les protéines du derme. Les composés selon l'invention peuvent ainsi être utilisés pour diminuer la glycation du collagène. Par ce terme, on entend tout type de collagène présent dans la peau.

Selon l'invention, l'ergothionéine et/ou de ses dérivés peuvent ainsi être utilisés :
- pour prévenir ou traiter les signes du vieillissement de la peau liés à la glycation, en particulier pour prévenir ou traiter la perte de tonicité de la peau due à l'âge ; et/ou
- pour amincir et/ou affiner la silhouette et/ou les contours du visage ; et/ou
- pour prévenir ou traiter l'aspect peau d'orange.

Au sens de la présente invention, l'ergothionéine et/ou ses dérivés répondent à la formule (I): 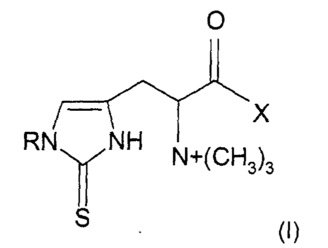 dans laquelle :
X représente un groupement -O-R' ou -NR'R";
R, R', R" représentent simultanément ou indépendamment l'un de l'autre, un atome d'hydrogène, un groupement alkyle, un groupement alkényle ou un groupement acyle en C₁-C₁₈, ces groupements étant linéaires ou ramifiés, éventuellement substitués par :
   (a) un groupement hydroxyle éventuellement estérifié, et/ou
   (b) un atome d'halogène, et/ou
   (c) un groupement carboxylique, et/ou
   (d) un groupement amine.

Selon une forme d'exécution préférée de l'invention, R désigne un atome d'hydrogène ou un groupement éthyle ou acétyle et X désigne NH₂. En particulier, on préfère utiliser l'ergothionéine, de préférence la L-(+)-ergothionéine.

La composition selon l'invention est de préférence destinée à un usage cosmétique ou dermatologique, avantageusement cosmétique. Elle est avantageusement destinée à une application topique sur la peau et contient donc généralement un milieu physiologiquement acceptable, c'est-à-dire compatible avec la peau.

Dans cette composition, l'ergothionéine et/ou son dérivé est de préférence présent en une quantité allant de 0,001% à 10% du poids total de la composition, mieux, en une quantité allant de 0,005.% à 5 % du poids total de la composition.

Cette composition peut se présenter sous toutes les formes galéniques normalement utilisées dans les domaines cosmétique et dermatologique, et elle peut être notamment sous forme d'une solution aqueuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, en particulier sous forme de stick. Elle peut être utilisée comme produit de soin et/ou comme produit de maquillage pour la peau.

De façon connue, la composition de l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse, dans les vésicules lipidiques et/ou dans les nanoparticules. En tout état de cause, ces adjuvants, ainsi que leurs proportions, seront choisis de manière à ne pas nuire aux propriétés recherchées de l'association d'actifs selon l'invention.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-20, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Pour une utilisation en vue de prévenir ou traiter la perte de tonicité de la peau et/ou d'affiner les contours du visage, l'ergothionéine peut être associée à d'autres actifs anti-âge, en particulier des actifs anti-rides, dépigmentants, raffermissants, drainants, tenseurs, anti-radicalaires et/ou immunoprotecteurs et/ou à des agents stimulant le métabolisme cellulaire (notamment la synthèse des protéines) et/ou la prolifération cellulaire.

Pour une utilisation en vue d'affiner la silhouette et/ou de prévenir ou traiter l'aspect peau d'orange, l'ergothionéine peut être associée à des actifs raffermissants, drainants, astringents, lipolytiques, tenseurs et/ou desquamants.

En cas d'incompatibilité, certains au moins des actifs mentionnés ci-dessus peuvent être incorporés dans des sphérules, notamment des vésicules ioniques ou non-ioniques et/ou des nanoparticules (nanocapsules et/ou nanosphères), de manière à ce que les actifs incompatibles entre eux soient isolés les uns des autres dans la composition.

L'invention sera mieux comprise, et ses avantages ressortiront mieux, à la lumière des exemples suivants, qui sont donnés à titre illustratif, et sans limitation.

### EXEMPLES

### Exemple 1 : Etude de l'effet de l'ergothionéine sur la glycation

La formation des produits de glycation est observée par la mise en présence d'une albumine sérique bovine (BSA) avec du D-ribose en absence ou en présence d'ergothionéine. L'inhibition de la glycation est mesurée par la variation de fluorescence émise.

Précisément, une solution de sérum albumine bovine à 1 mg/ml en solution dans du tampon phosphate salin (PBS) est incubée à 37°C pendant 14 jours avec du D-ribose à la concentration 10mM, en présence ou en absence d'ergothionéine aux concentrations de 500 µM, 1 mM ou 5 mM.

La glycation est évaluée en mesurant la fluorescence des AGEs à λem. = 440 nm émise par chaque échantillon après excitation à λex. = 370 nm. L'inhibition de la glycation est visualisée par la diminution de la fluorescence comparée à l'échantillon traité avec le sucre seul.

Les résultats sont rassemblés dans le Tableau 1 ci-dessous et exprimés en pourcentage d'inhibition de la fluorescence par rapport au témoin glyqué :

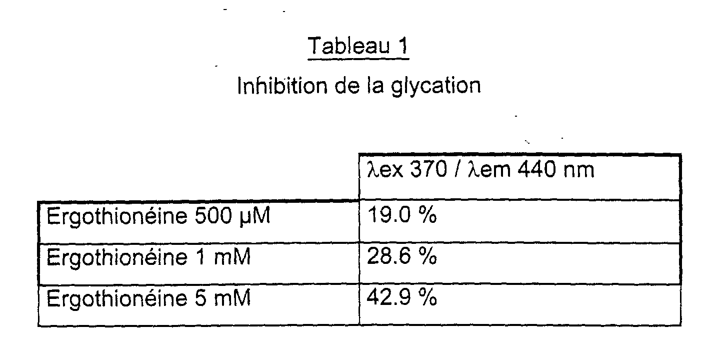

L'Ergothionéine semble ainsi posséder une activité anti-glycation intéressante, dépendante de la concentration d'actif.

### Exemple 2 : Compositions cosmétiques

Les quantités des constituants des compositions données à titre illustratif ci-après sont indiquées en pourcentage pondéral. Ces compositions sont préparées selon les techniques usuelles bien connues de l'homme du métier.

| Exemple 2-1 : Crème anti-âge | |
|---|---|
| Ergothionéine | 1 % |
| Tristéarate de sorbitanne | 0,9 % |
| Stéarate de polyéthylène glycol (40 OE) | 2 % |
| Alcool cétylique | 4 % |
| Stéarate de glycéryle | 3 % |
| Acide stéarique | 1,2 % |
| Vaseline | 4 % |
| Polyisobutène hydrogéné | 8,5 % |
| Myristate de myristyle | 2 % |
| Cyclopentasiloxane | 5 % |
| Glycérine | 3 % |
| Conservateurs | 0,3 % |
| Hydroxyde de sodium | 0,05% |
| Eau | qsp. 100 % |

| Exemple 2-2 : Composition amincissante | |
|---|---|
| Ergothionéine | 1 % |
| Copolymère PVM/MA décadiène | 0,45 % |
| Cyclohexasiloxane | 7 % |
| Carbomer | 0,3 % |
| Triéthanolamine | 0,5 % |
| Propylène glycol | 3 % |
| Caféine | 2 % |
| Gélifiant (Sepigel 305 de la société SEPPIC) | 0,25 % |
| Eau | qsp. 100 % |

| Exemple 2-3 : Crème anti-peau d'orange | |
|---|---|
| Ergothionéine | 1 % |
| Sodium diméthicone copolyol acétyl méthyltaurate | |
| (Pecosil DCT de la société PHOENIX) | 5,4 % |
| Huile minérale | 19 % |
| Glycérine | 5 % |
| Conservateurs | 0,25 % |
| Caféine | 1 % |
| Gomme de xanthane | 0,25% |
| Sel d'ammonium de l'acide acrylamidométhylpropane sulfonique | 1,2 % |
| Mélange de cyclopentasiloxane et diméthiconol | 5 % |
| Extrait de Gingko biloba dans le propylène glycol | 5 % |
| Eau | qsp. 100 % |

## Revendications

1. Utilisation d'ergothionéine et/ou de ses dérivés dans une composition cosmétique, en tant qu'agent pour diminuer la glycation des protéines de la peau.

2. Utilisation d'ergothionéine et/ou de ses dérivés pour la fabrication d'une composition dermatologique destinée à diminuer la glycation des protéines de la peau.

3. Utilisation selon la revendication 1 ou 2, **caractérisée par le fait que** les protéines de la peau sont les protéines du derme.

4. Utilisation selon la revendication précédente, pour diminuer la glycation du collagène.

5. Utilisation d'ergothionéine et/ou de ses dérivés pour prévenir ou traiter les signes du vieillissement de la peau liés à la glycation.

6. Utilisation d'ergothionéine et/ou de ses dérivés pour prévenir ou traiter la perte de tonicité de la peau due à l'âge.

7. Utilisation d'ergothionéine et/ou de ses dérivés pour amincir et/ou affiner la silhouette et/ou les contours du visage.

8. Utilisation d'ergothionéine et/ou de ses dérivés pour prévenir ou traiter l'aspect peau d'orange.

9. Utilisation selon l'une quelconque des revendications précédentes, **caractérisée par le fait que** l'ergothionéine et/ou ses dérivés répondent à la formule (I): 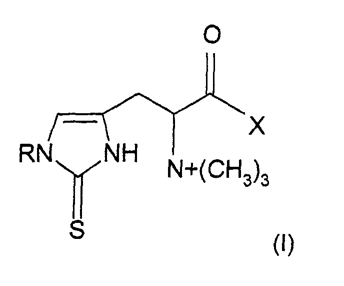 dans laquelle :
X représente un groupement -O-R'ou -NR'R";
R, R', R" représentent simultanément ou indépendamment l'un de l'autre, un atome d'hydrogène, un groupement alkyle, un groupement alkényle ou un groupement acyle en C₁-C₁₈, ces groupements étant linéaires ou ramifiés, éventuellement substitués par :
(a) un groupement hydroxyle éventuellement estérifié, et/ou
(b) un atome d'halogène, et/ou
(c) un groupement carboxylique, et/ou
(d) un groupement amine.

10. Utilisation selon la revendication 9, **caractérisée par le fait que** R désigne un atome d'hydrogène ou un groupement éthyle ou acétyle et X désigne NH₂.

11. Utilisation selon la revendication 10, **caractérisée en ce que** le composé de formule (I) est l'ergothionéine.

12. Utilisation selon la revendication 11, **caractérisée en ce que** le composé de formule (I) est la L-(+)-ergothionéine.

13. Utilisation selon l'une quelconque des revendications précédentes, **caractérisée par le fait que** la composition est destinée à une application topique sur la peau.

14. Utilisation selon l'une quelconque des revendications précédentes, **caractérisée par le fait que** la composition renferme en outre un milieu physiologiquement acceptable.

15. Utilisation selon l'une quelconque des revendications précédentes, **caractérisée par le fait que** l'ergothionéine et/ou son dérivé est présent en une quantité allant de 0,001% à 10% du poids total de la composition.

16. Utilisation selon la revendication précédente, **caractérisée par le fait que** l'ergothionéine et/ou son dérivé est présent en une quantité allant de 0,005.% à 5 % du poids total de la composition.
